# EUROPEAN PATENT SPECIFICATION

(11) **EP 1 679 380 B1**
(45) Date of publication and mention of the grant of the patent: **27.10.2010**
(21) Application number: 06000412.4
(22) Date of filing: 10.01.2006
(51) Int. Cl.: C12Q 1/68

(54) **Methods for detecting bacteria of the genus Mycobacterium (acid-fast bacteria)**
Verfahren zum Nachweis von Bakterien der Gattung Mycobacterium (säureresistente Bakterien)
Méthodes pour la détection de bactéries du genre Mycobacterium (bactéries acido-résistantes)

(30) Priority: 11.01.2005 JP 2005003493; 15.04.2005 JP 2005117816
(43) Date of publication of application: 12.07.2006
(73) Proprietor: FUJIFILM Corporation, Minato-ku Tokyo (JP)
(72) Inventor: Iwaki, Yoshihide, Fuji Photo Film Co., Ltd., Asaka-shi, Saitama 351-8585 (JP)
(74) Representative: Hiebl, Inge Elisabeth

(56) References cited:
- EP-A- 1 431 400
- WO-A-01/31062
- OGGIONI M R ET AL: "Identification of Mycobacterium tuberculosis complex, Mycobacterium avium and Mycobacterium intracellulare by selective nested polymerase chain reaction." MOLECULAR AND CELLULAR PROBES. OCT 1995, vol. 9, no. 5, October 1995 (1995-10), pages 321-326, XP008063897 ISSN: 0890-8508
- KIRSCHNER P ET AL: "GENOTYPIC IDENTIFICATION OF MYCOBACTERIA BY NUCLEIC ACID SEQUENCE DETERMINATION: REPORT OF A 2-YEAR EXPERIENCE IN A CLINICAL LABORATORY" JOURNAL OF CLINICAL MICROBIOLOGY, WASHINGTON, DC, US, vol. 31, no. 11, November 1993 (1993-11), pages 2882-2889, XP009016331 ISSN: 0095-1137
- HALL GERRI S ET AL: "Molecular mycobacteriology" JOURNAL OF CLINICAL LIGAND ASSAY, vol. 27, no. 3, October 2004 (2004-10), pages 205-211, XP008063920 ISSN: 1081-1672
- SHRESTHA N K ET AL: "Detection and differentiation of Mycobacterium tuberculosis and nontuberculous mycobacterial isolates by real-time PCR" JOURNAL OF CLINICAL MICROBIOLOGY, WASHINGTON, DC, US, vol. 41, no. 11, November 2003 (2003-11), pages 5121-5126, XP002370873 ISSN: 0095-1137
- TUOHY M J ET AL: "Pyrosequencing(TM) as a tool for the identification of common isolates of Mycobacterium sp" DIAGNOSTIC MICROBIOLOGY AND INFECTIOUS DISEASES, ELSEVIER SCIENCE PUBLISHING CO., AMSTERDAM, NL, vol. 51, no. 4, April 2005 (2005-04), pages 245-250, XP004830212 ISSN: 0732-8893

### SEQUENCE LISTING

<110> Fuji Photo Film Co.Ltd.,
<120> A method for detection of Mycobacterium spp and a kit therefor
<130> FA5160A
<160> 28
<210> 1
   <211> 44
   <212> DNA
   <213> Mycobacterium tuberculosis
<400> 1
   gataggacca cgggatgcat gtcttgtggt ggaaagcgct ttag 44
<210> 2
   <211> 18
   <212> DNA
   <213> Mycobacterium tuberculosis
<400> 2
   acgggatgca tgtcttgt 18
<210> 3
   <211> 69
   <212> DNA
   <213> Mycobacterium tuberculosis
<400> 3
   gcctgggaaa ctgggtctaa taccggatag gaccacggga tgcatgtctt gtggtggaaa 60
   gcgctttag 69
<210> 4
   <211> 42
   <212> DNA
   <213> Mycobacterium avium
<400> 4
   gataggacct caagacgcat gtcttctggt ggaaagcttt tg 42
<210> 5
   <211> 42
   <212> DNA
   <213> Mycobacterium avium
<400> 5
   tcaagacgca tgtcttct 18
<210> 6
   <211> 67
   <212> DNA
   <213> Mycobacterium avium
<400> 6
   gcctgggaaa ctgggtctaa taccggatag gacctcaaga cgcatgtctt ctggtggaaa 60
   gcttttg 67
<210> 7
   <211> 42
   <212> DNA
   <213> Mycobacterium intracellulare
<400> 7
   gataggacct ttaggcgcat gtctttaggt ggaaagcttt tg 42
<210> 8
   <211> 18
   <212> DNA
   <213> Mycobacterium intracellulare
<400> 8
   tttaggcgca tgtcttta 18
<210> 9
   <211> 67
   <212> DNA
   <213> Mycobacterium intracellulare
<400> 9
   gcctgggaaa ctgggtctaa taccggatag gacctttagg cgcatgtctt taggtggaaa 60
   gcttttg 67
<210> 10
   <211> 18
   <212> DNA
   <213> Artificial Sequence
<220>
   <223> Description of Artificial Sequence: Synthetic DNA
<400> 10
   ataccggata ggaccacg 18
<210> 11
   <211> 18
   <212> DNA
   <213> Artificial Sequence
<220>
   <223> Description of Artificial Sequence: Synthetic DNA
<400> 11
   ataccggata ggacctca 18
<210> 12
   <211> 19
   <212> DNA
   <213> Artificial Sequence
<220>
   <223> Description of Artificial Sequence: Synthetic DNA
<400> 12
   aataccggat aggaccttt 19
<210> 13
   <211> 19
   <212> DNA
   <213> Artificial Sequence
<220>
   <223> Description of Artificial Sequence: Synthetic DNA
<400> 13
   tgcttcttct ccacctacc 19
<210> 14
   <211> 16
   <212> DNA
   <213> Artificial Sequence
<220>
   <223> Description of Artificial Sequence: Synthetic DNA
<400> 14
   taccggatag gaccac 16
<210> 15
   <211> 19
   <212> DNA
   <213> Artificial Sequence
<220>
   <223> Description of Artificial Sequence: Synthetic DNA
<400> 15
   ataccggata ggacctcaa 19
<210> 16
   <211> 17
   <212> DNA
   <213> Artificial Sequence
<220>
   <223> Description of Artificial Sequence: Synthetic DNA
<400> 16
   taccggatag gacctca 17
<210> 17
   <211> 18
   <212> DNA
   <213> Artificial Sequence
<220>
   <223> Description of Artificial Sequence: Synthetic DNA
<400> 17
   taccggatag gacctcaa 18
<210> 18
   <211> 19
   <212> DNA
   <213> Artificial Sequence
<220>
   <223> Description of Artificial Sequence: Synthetic DNA
<400> 18
   ataccggata ggaccttta 19
<210> 19
   <211> 18
   <212> DNA
   <213> Artificial Sequence
<220>
   <223> Description of Artificial Sequence: Synthetic DNA
<400> 19
   taccggatag gaccttta 18
<210> 20
   <211> 18
   <212> DNA
   <213> Artificial Sequence
<220>
   <223> Description of Artificial Sequence: Synthetic DNA
<400> 20
   cggataggac cacgggat 18
<210> 21
   <211> 21
   <212> DNA
   <213> Artificial Sequence
<220>
   <223> Description of Artificial Sequence: Synthetic DNA
<400> 21
   taccggatag gacctcaaga c 21
<210> 22
   <211> 21
   <212> DNA
   <213> Artificial Sequence
<220>
   <223> Description of Artificial Sequence: Synthetic DNA
<400> 22
   ataccggata ggacctttag g 21
<210> 23
   <211> 45
   <212> DNA
   <213> Mycobacterium kansasii
<400> 23
   gataggacca cttggcgcat gccttgtggt ggaaagcttt tgcgg 45
<210> 24
   <211> 18
   <212> DNA
   <213> Mycobacterium kansasii
<400> 24
   acttggcgca tgccttgt 18
<210> 25
   <211> 70
   <212> DNA
   <213> Mycobacterium kansasii
<400> 25
   gcctgggaaa ctgggtctaa taccggatag gaccacttgg cgcatgcctt gtggtggaaa 60
   gcttttgcgg 70
<210> 26
   <211> 20
   <212> DNA
   <213> Artificial Sequence
<220>
   <223> Description of Artificial Sequence: Synthetic DNA
<400> 26
   ataccggata ggaccacttg 20
<210> 27
   <211> 19
   <212> DNA
   <213> Artificial Sequence
<220>
   <223> Description of Artificial Sequence: Synthetic DNA
<400> 27
   taccggatag gaccacttg 19
<210> 28
   <211> 18
   <212> DNA
   <213> Artificial Sequence
<220>
   <223> Description of Artificial Sequence: Synthetic DNA
<400> 28
   cgtcctgtgc atgtcaaa 18

## Description

### TECHNICAL FIELD

The present invention relates to an oligonucleotide for rapidly and conveniently detecting bacteria of the genus Mycobacterium (acid-fast bacteria) or for identifying the bacterial species thereof, and a method for detecting bacteria of the genus Mycobacterium (acid-fast bacteria) using such oligonucleotide.

### BACKGROUND ART

In Japan, tuberculosis has been one of the major causes of death for many years. However, the number of patients with tuberculosis has drastically decreased because of improved living environments, better hygiene, and advanced chemotherapy. Even today, eight million patients with tuberculosis occur annually in the whole world and about three million people die from the disease every year. Currently, there is concern about a possible mass infection of young people having no immunity to tuberculosis. Furthermore, there is concern that carriers who have become infected with *Tubercle bacillus* during epidemic seasons could suddenly develop tuberculosis as they age and decrease in physical strength. Furthermore, infectious diseases due to bacteria referred to as atypical acid-fast bacteria are on the increase. In particular, the *Mycobacterium avium* complex (MAC) infectious disease is intractable and is problematic as an opportunistic infectious disease impacting AIDS patients.

Therefore, diagnosis and treatment for tuberculosis and atypical mycobacteriosis are clinically very important: The symptoms arising from human *Tubercle bacillus* (*Mycobacterium tuberculosis*) are very severe. Antibiotics such as streptomycin, rifampicin, and ethambutol are effective against *Tubercle bacillus* and treatment should be initiated early. The source of infection is a patient, and *Tubercle bacillus* infection occurs via the airway, such as by droplet infection. Thus, early diagnosis is also important for suppressing epidemics. The disease images of atypical mycobacteriosis have no specificities, and the effects of chemotherapy against the disease differ depending on the bacterial species. Hence, early diagnosis and treatment are needed.

*Tubercle bacillus* has been conventionally tested by culture methods. In general, separation and culture are performed using Ogawa media and then bacterial species are identified based on properties (e.g., growth rate, temperature, colony shape, and pigment production) appearing on media and biochemical properties determined by a niacin test, a nitrate reduction test, a thermostable catalase test, a Tween 80 hydrolysis test, or the like. However, acid-fast bacteria grow slowly, so that 1 or more months are required to conduct the above tests.

Moreover, a method for detecting protein produced by human *Tubercle bacillus* by an antigen-antibody reaction, has also been developed. However, the method is problematic in terms of sensitivity, so that it still requires culturing of bacteria.

Recently, rapid identification of bacteria using genes has been developed. Such techniques are also applied for detection and identification of *Tubercle bacillus* and acid-fast bacteria. For example, "AccuProbe" (KYOKUTO PHARMACEUTICAL INDUSTRIAL CO., LTD.) and "DDH Mycobacterium" (KYOKUTO PHARMACEUTICAL INDUSTRIAL CO., LTD.) have been developed as kits for identifying bacterial strains using nucleic acids. However, these kits still require culturing of bacteria.

As kits for identifying bacterial strains that do not require culturing of bacteria, "DNA probe "RG"-MTD" (FUJIREBIO INC.), "AMPLICOR Mycobacterium" (Roche Diagnostics), and the like using a nucleic acid amplification method have been developed. By the use of these kits, *Tubercle bacillus* can be detected and identified within approximately 1 day from a clinical specimen such as sputum.

However, these gene diagnosis methods also involve problems. These kits enable detection and identification within 1 day. However, in view of needs at actual clinical sites, it is preferable to obtain the results during time period ranging from the arrival of a patient at the hospital to his or her departure from the hospital. Specifically, such duration may be within half a day. Therefore, further acceleration of diagnosis is required.

Furthermore, a detection system using chemiluminescence or a large-scale automatic machine are also problematic in that initial equipment investment and cost per test are excessively expensive. Accordingly, testing at low cost is an important issue surrounding gene diagnosis methods.

Oggioni M R et al. "Identification of Mycobacterium tuberculosis complex, Mycoabcterium avium and Mycobacterium intracellulare by selective nested polymerase chain reaction", Molecular and Cellular Probes, vol. 9, no. 5, October 1995 (1995-10), pages 321-326, discloses a nested polymerase chain reaction (PCR procedure) for identification of Mycobacteria exploiting genus-specific sequences on the 16S rRNA.

Kirschner P et al. "Genotypic identification of mycobacteria by nucleic acid sequence determination: report of a 2-year experience in a clinical laboratory", Journal of Clinical Microbiology, vol. 31, no. 11, November 1993 (1993-11), pages 2882-2889, describes the identification of clinical isolates of Mycobacterium spp. by direct sequence determination of 16S rRNA gene fragments amplified by polymerase chain reaction. The identification was based on a hypervariable region within the 16S rRNA gene, in which mycobacterial species are characterized by species-specific nucleotide sequences.

Hall Gerri S et al. "Molecular mycobacteriology", Journal of Clinical Ligand Assay, vol. 27, no. 3, October 2004 (2004-10), pages 205-211, discusses the methods that are presently available commercially or in research formats for the direct molecular approaches to the identification of *Mycobacterium* sp. in clinical specimens or from cultured isolates.

EP 1 431 400 discloses a method of detecting a specific acid-fast bacterium, wherein primers having nucleotide sequences homologous or complementary to the specific regions in the rRNAs of specific acid-fast bacteria are used to specifically amplify only the rRNAs of the specific acid-fast bacterium.

Shrestha N K et al. "Detection and differentiation of Mycobacterium tuberculosis and nontuberculous mycobacterial islates by real-time PCR", Journal of Clinical Microbiology, vol. 41, no. 11, November 2003 (2003-11), pages 5121-5126, describes a real-time PCR for Mycobacteria by using the LightCycler system with 18 reference strains and 168 clinical mycobacterial isolates.

WO 01/31062 relates to homogenous detection assay formats for detecting variants of short nucleotide sequences.

Tuohy M J et al. "Pyrosequencing™ as a tool for the identification of common isolates of Mycobacterium sp.", Diagnostic Microbiology and Infectious Diseases, vol. 51, no. 4, April 2005 (2005-04), pages 245-250, discloses pyrosequencing of hypervariable regions of Mycobacterium as a rapid and acceptable tool for the characterization of common routinely isolated clinical Mycobacterium species.

### DISCLOSURE OF THE INVENTION

An object of the present invention is to provide an oligonucleotide for rapidly and conveniently detecting bacteria of the genus Mycobacterium (acid-fast bacteria) or for identifying the bacterial species thereof, and a method and kit for detecting bacteria of the genus Mycobacterium (acid-fast bacteria) using such oligonucleotide.

As a result of intensive studies concerning a 16S rRNA (ribosomal RNA) gene of bacteria of the genus Mycobacterium (acid-fast bacteria) for the purpose of achieving the above object, the present inventors have discovered nucleotide sequences appropriate for detecting bacteria of the genus Mycobacterium (acid-fast bacteria) or for identifying the bacterial species thereof. Thus the present inventors have succeeded in establishment of a detection method using the same and have completed the present invention.

Thus, the present invention provides a method for identifying *Mycobacterium tuberculosis,* which comprises performing a nucleic acid amplification reaction using as primers an upper primer having a nucleotide sequence represented by SEQ ID NO: 10 and a lower primer represented by SEQ ID NO: 13.

Further, the present invention provides a method for identifying *Mycobacterium avium,* which comprises performing a nucleic acid amplification reaction using as primers an upper primer having a nucleotide sequence represented by SEQ ID NO: 11 and a lower primer represented by SEQ ID NO: 13.

Further, the present invention provides a method for identifying *Mycobacterium intracellulare,* which comprises performing a nucleic acid amplification reaction using as primers an upper primer having a nucleotide sequence represented by SEQ ID NO: 12 and a lower primer represented by SEQ ID NO: 13.

Further, the present invention provides a method for identifying *Mycobacterium kansasii,* which comprises performing a nucleic acid amplification reaction using as primers an upper primer having a nucleotide sequence represented by SEQ ID NO: 26 and a lower primer represented by SEQ ID NO: 28.

Preferably, a by-product of a nucleic acid amplification reaction can be detected.

Preferably, the by-product of the nucleic acid amplification reaction is pyrophosphoric acid.

Preferably, pyrophosphoric acid is detected using a dry analytical element. Further, described is a primer for nucleic acid amplification for use in identification of *Mycobacterium tuberculosis,* whicb has a nucleotide sequence represented by SEQ ID NO: 10, 14 or 20.

Further, described is a primer for nucleic acid amplification for use in identification of *Mycobacterium avium,* which has a nucleotide sequence represented by SEQ ID NO: 11, 15, 16, 17 or 21.

Further, described is a primer for nucleic acid amplification for use in identification of *Mycobacterium intracellulare,* which has a nucleotide sequence represented by SEQ ID NO: 12, 18, 19 or 22.

Further, described is a primer for nucleic acid amplification for use in identification of *Mycobacterium kansasii,* which has a nucleotide sequence represented by SEQ ID NO: 26 or 27.

Further, described is a kit for detecting the genus Mycobacterium (acid-fast bacteria), which contains at least 1 type of primer for nucleic acid amplification as mentioned above, at least 1 type of deoxynucleoside triphosphate, at least 1 type of polymerase, and a dry analytical element.

### BEST MODE FOR CARRYING OUT THE INVENTION

Embodiments of the present invention will be described in detail as follows.

The method for detecting the genus Mycobacterium (acid-fast bacteria) of the present invention comprises the use of primers having sequences specific to each bacterial species at the 3' end. By the use of the method of the present invention, the genus Mycobacterium (acid-fast bacteria) can be identified. In a preferred embodiment of the method of the present invention, nucleic acid amplification reaction is performed using primers specific to each bacterium, thereby detecting the presence or the absence of the relevant extension reaction. Specific examples of detection methods include methods that involve directly measuring amplification products such as electrophoresis, mass spectrometry and liquid chromatography, and methods that involve detecting pyrophosphoric acid generated upon a polymerase elongation reaction.

Whether or not elongation reaction has actually taken place is preferably confirmed by detection of pyrophosphoric acid. More preferably, pyrophosphoric acid can be detected using a dry analytical element for quantifying pyrophosphoric acid, which is provided with a reagent layer containing xanthosine or inosine, pyrophosphatase, purine nucleoside phosphorylase, xanthine oxidase, peroxidase, and a color developer. The use of such dry analytical element for quantifying pyrophosphoric acid enables detection within 5 minutes.

### (A) Primer for nucleic acid amplification used in the present invention

The primers used are as described above.

### (B) Nucleic acid amplification method

For nucleic acid amplification performed as per the method of the present invention, various methods that have been developed can be used. Examples of methods for nucleic acid amplification that can be used in the present invention include PCR (JP Patent Publication (Kokoku) No. 4-67960 B (1992) and Patent Publication (Kokoku) No. 4-67957 B (1992)), LCR (JP Patent Publication (Kokai) No. 5-2934 A (1993)), SDA (Strand Displacement Amplification: JP Patent Publication (Kokai) No. 5-130870 A (1993)), RCA (Rolling Circle Amplification: Proc. Natl. Acad. Sci, Vol.92, 4641-4645 (1995)), ICAN (Isothermal and Chimeric Primer-initiated Amplification of Nucleic Acids), LAMP (Loop-Mediated Isothermal Amplification of DNA: Bio Industry, vol. 18, No. 2 (2001)), NASBA (Nucleic Acid Sequence-based Amplification Method: Nature, 350, 91 (1991)), and TMA (Transcription Mediated Amplification Method: J. Clin Microbiol. vol. 31, 3270 (1993)).

The most generally and widely used method among the above methods for nucleic acid amplification is the PCR (polymerase chain reaction) method. In the PCR method, periodic steps are repeated by periodic control of increases and decreases in the temperature of a reaction solution. Specifically, the periodic steps are: denaturation (the step of denaturing a nucleic acid fragment from double strands into single strands) → annealing (the step of causing a primer to hybridize to the denatured single strand of the nucleic acid fragment) → polymerase (Taq DNA polymerase) elongation reaction → denaturation. Thus an objective portion of a target nucleic acid fragment is amplified. Finally, the objective portion of a target nucleic acid fragment can be amplified to an amount one million times greater than the initial amount.

In the LCR (JP Patent Publication (Kokai) No. 5-2934 A (1993)) method, two complementary oligonucleotide probe strands are bound end-to-tail to a single-stranded DNA so as to fill the nicks between two oligonucleotide strands by thermostable ligase. The thus-bound DNA strand is liberated by denaturation. Amplification is then performed using the liberated strand as a template. SNP determination is possible based on the presence or the absence of amplification by contriving probe sequences. Furthermore, a method has also been developed by improving LCR; specifically, by providing gaps between two primers and then by filling in the gaps by polymerase (Gap-LCR: Nucleic Acids Research, vol. 23, No. 4, 675 (1995)).

The SDA (Strand Displacement Amplification: JP Patent Publication (Kokai) No. 5-130870 A (1993)) method is a cycling assay method using exonuclease. Specifically, the SDA method is one of methods for amplifying an objective site of a target nucleic acid fragment using a polymerase elongation reaction. In this method, 5' → 3'exonuclease is caused to act simultaneously with a polymerase elongation reaction that uses as a starting point a primer specifically hybridizing to an objective site of a target nucleic acid fragment, thereby degrading the primer from the opposite direction. A new primer hybridizes in place of the degraded primer, so that elongation reaction proceeds again by DNA polymerase. Such elongation reaction that is performed by polymerase and degradation reaction that is performed by exonuclease so as to remove the previously extended strand are periodically repeated in order. Here, the elongation reaction by polymerase and the degradation reaction by exonuclease can be implemented under isothermal conditions.

The LAMP method is a recently developed method for amplifying an objective site of a target nucleic acid fragment. The LAMP method is a method for amplifying as a special structure an objective site of a target nucleic acid fragment under isothermal conditions. Specifically, the LAMP method is performed using at least 4 types of primer that complementarily recognize specific sites at at least 6 positions in a target nucleic acid fragment and strand displacement type Bst DNA polymerase that lacks 5' → 3'nuclease activity and catalyzes an elongation reaction while liberating double-stranded DNA on a template in the form of single-stranded DNA.

The ICAN method is also a recently developed method for amplifying an objective site of a target nucleic acid fragment. The ICAN method is an isothermal gene amplification method using RNA-DNA chimeric primers, DNA polymerase having strand displacing activity and template switching activity, and RNaseH. After chimeric primers bind to a template, a complementary strand is synthesized by DNA polymerase. Subsequently, RNaseH cleaves RNA portions derived from the chimeric primers and then an elongation reaction takes place together with a strand displacement reaction and a template switching reaction from the cleaved portions. The gene is amplified by such reaction, which takes place repeatedly.

### (C) Detection

The method of the present invention uses primers for nucleic acid amplification, by which bacterial species can be identified. Hence, detection means is not specifically limited, as long as the means enables quantification of the amounts of amplification products.

Examples of detection methods include methods that involve directly measuring generated products such as electrophoresis, liquid chromatography or mass spectrometer, and methods for detecting pyrophosphoric acid or the like that is generated as a result of polymerase reaction. In view of quantification ability, a detection method for quantifying pyrophosphoric acid is preferable. In view of convenience, a detection method for quantifying pyrophosphoric acid using a dry analytical element is more preferable.

A method represented by formula 1 has been heretofore known as a method for detecting pyrophosphoric acid (PPi). In this method, pyrophosphoric acid (PPi) is converted into adenosinetriphosphate (ATP) with the aid of sulfurylase, and luminescence generated when adenosinetriphosphate acts on luciferin with the aid of luciferase is detected. Thus, an apparatus capable of measuring luminescence is required for detecting pyrophosphoric acid (PPi) by this method.

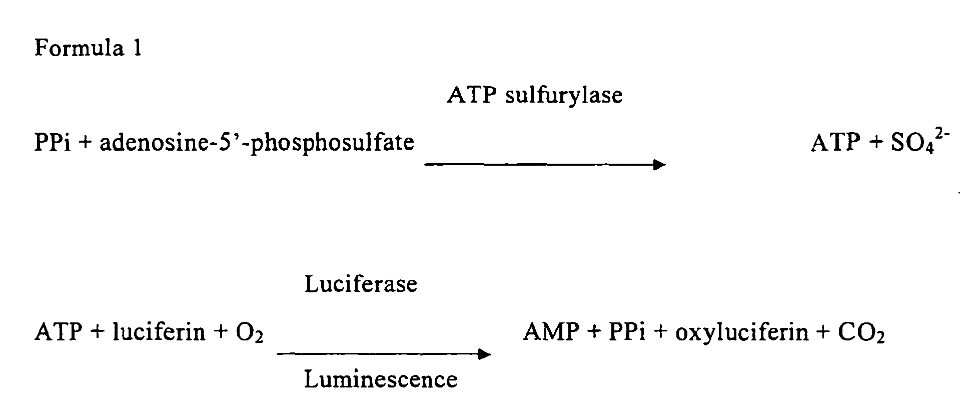

A method for detecting pyrophosphoric acid suitable for the present invention is a method represented by formula 2 or 3. In the method represented by formula 2 or 3, pyrophosphoric acid (PPi) is converted into inorganic phosphate (Pi) with the aid of pyrophosphatase, inorganic phosphate (Pi) is reacted with xanthosine or inosine with the aid of purine nucleoside phosphorylase (PNP), the resulting xanthine or hypoxanthine is oxidated with the aid of xanthine oxidase (XOD) to generate uric acid, and a color developer (a dye precursor) is allowed to develop color with the aid of peroxidase (POD) using hydrogen peroxide (H₂O₂) generated in the oxidation process, followed by colorimetry. In the method represented by formula 2 or 3, the result can be detected by colorimetry and, thus, pyrophosphoric acid (PPi) can be detected visually or using a simple colorimetric measuring apparatus.

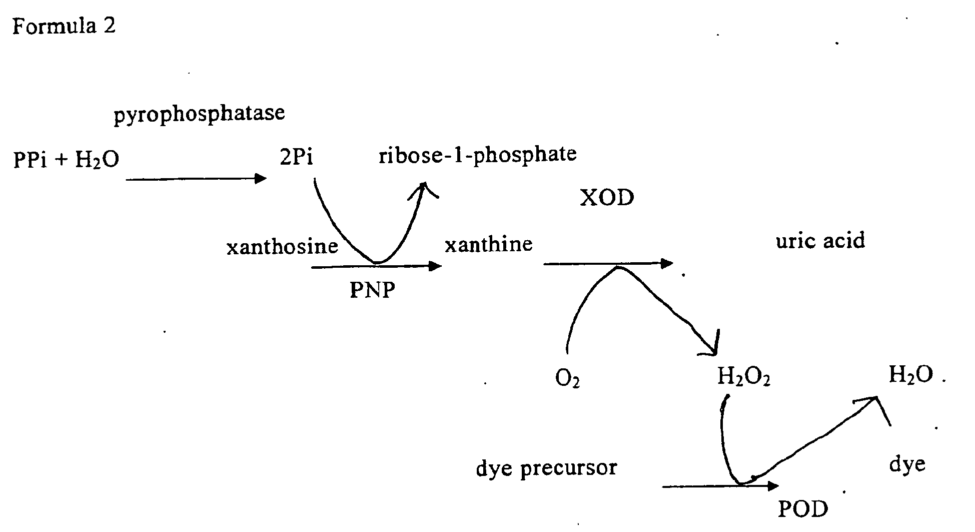

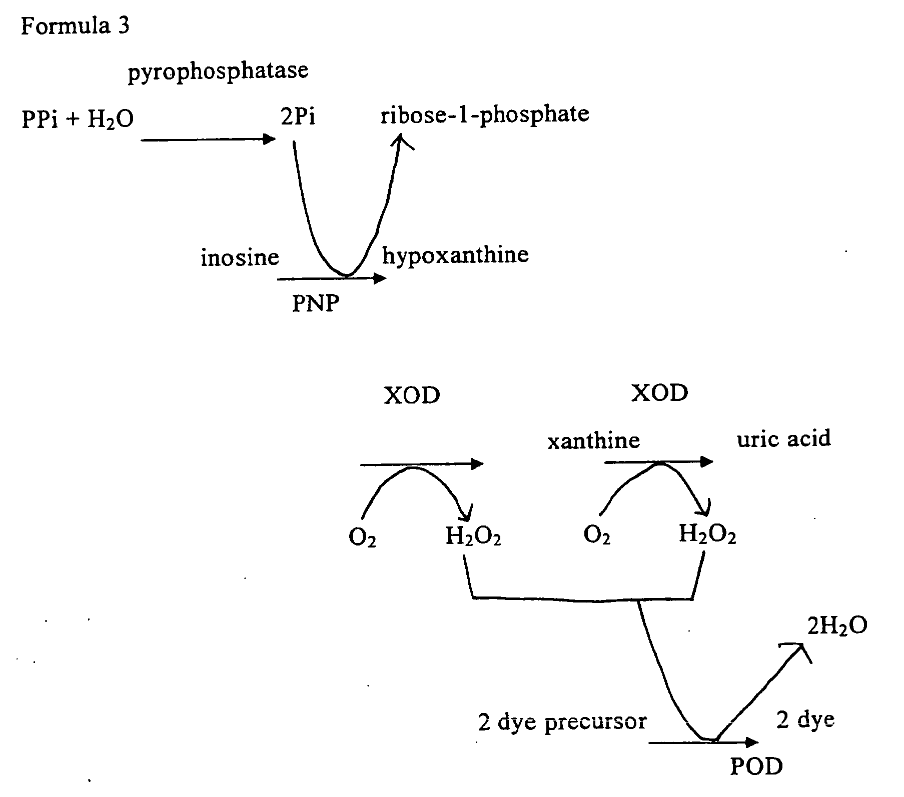

Commercially available pyrophosphatase (EC3, 6, 1, 1), purine nucleoside phosphorylase (PNP, EC2. 4. 2. 1), xanthine oxidase (XOD, EC1. 2. 3. 2), and peroxidase (POD, EC1. 11. 1. 7) can be used. A color developer (i.e., a dye precursor) may be any one as long as it can generate a dye by hydrogen peroxide and peroxidase (POD), and examples thereof which can be used herein include: a composition which generates a dye upon oxidation of leuco dye (e.g., triarylimidazole leuco dye described in U.S.Patent. No. 4,089,747 and the like, diarylimidazole leuco dye described in Japanese Patent Publication Laying-Open No. 59-193352 (EP 0122641A)); and a composition (e.g., 4-aminoantipyrines and phenols or naphthols) containing a compound generating a dye by coupling with other compound upon oxidation.

### (D) Dry analytical element

A dry analytical element which can be used in the present invention is an analytical element which comprises a single or a plurality of functional layers, wherein at least one layer (or a plurality of layers) comprises a detection reagent, and a dye generated upon reaction in the layer is subjected to quantification by colorimetry by reflected light or transmitted light from the outside of the analytical element.

In order to perform quantitative analysis using such a dry analytical element, a given amount of liquid sample is spotted onto the surface of a developing layer. The liquid sample spread on the developing layer reaches the reagent layer and reacts with the reagent thereon and develops color. After spotting, the dry analytical element is maintained for a suitable period of time at given temperature (for incubation) and a color developing reaction is allowed to thoroughly proceed. Thereafter, the reagent layer is irradiated with an illuminating light from, for example, a transparent support side, the amount of reflected light in a specific wavelength region is measured to determine the optical density of reflection, and quantitative analysis is carried out based on the previously determined calibration curve.

Since a dry analytical element is stored and kept in a dry state before detection, it is not necessary that a reagent is prepared for each use. As stability of the reagent is generally higher in a dry state, it is better than a so-called wet process in terms of simplicity and swiftness since the wet process requires the preparation of the reagent solution for each use. It is also excellent as an examination method because highly accurate examination can be swiftly carried out with a very small amount of liquid sample.

### (E) Dry analytical element for quantifying pyrophosphoric acid

A dry analytical element for quantifying pyrophosphoric acid which can be used in the present invention can have a layer construction which is similar to various known dry analytical elements. The dry analytical element may be multiple layers which contain, in addition to a reagent for performing the reaction represented by formula 2 or 3 according to item (E) above (detection of pyrophosphoric acid (PPi)), a support, a developing layer, a detection layer, a light-shielding layer, an adhesive layer, a water-absorption layer, an undercoating layer, and other layers. Examples of such dry analytical elements include those disclosed in the specifications of Japanese Patent Publication Laying-Open No. 49-53888 (U.S. Patent. No. 3,992,158), Japanese Patent Publication Laying-Open No. 51-40191 (U.S. Patent. No. 4,042,335), Japanese Patent Publication Laying-Open No. 55-164356 (U.S.Patent. No. 4,292,272), and Japanese Patent Publication Laying-Open No. 61-4959 (EPC Publication No. 0166365A).

Examples of the dry analytical element to be used in the present invention include a dry analytical element for quantifying pyrophosphoric acid which comprises a reagent for converting pyrophosphoric acid into inorganic phosphorus and a reagent layer containing a group of reagent for carrying out a coloring reaction depending of the amount of inorganic phosphorus.

In this dry analytical element for quantitative assay of pyrophosphate, pyrophosphoric acid (PPi) can enzymatically be converted into inorganic phosphorus (Pi) using pyrophosphatase as described above. The subsequent process, that is color reaction depending on the amount of inorganic phosphorus (Pi), can be performed using "quantitative assay method of inorganic phosphorus" (and combinations of individual reactions used therefor), described hereinafter, which is known in the field of biochemical inspection.

It is noted that when representing "inorganic phosphorus," both the expressions "Pi" and "HPO₄²⁻, H₂PO₄¹⁻" are used for phosphoric acid (phosphate ion). Although the expression "Pi" is used in the examples of reactions described below, the expression "HPO₄²⁻" may be used for the same reaction formula.

As the quantitative assay method of inorganic phosphorus, an enzyme method and a phosphomolybdate method are known. Hereinafter, this enzyme method and phosphomolybdate method will be described as the quantitative assay method of inorganic phosphorus.

### A. Enzyme method

Depending on the enzyme to be used for the last color reaction during a series of reactions for Pi quantitative detection, the following methods for quantitative assay are available: using peroxidase (POD); or using glucose-6-phosphate dehydrogenase (G6PDH), respectively. Hereinafter, examples of these methods are described.

### (1) Example of the method using peroxidase (POD)

### (1-1)

Inorganic phosphorus (Pi) is allowed to react with inosine by purine nucleoside phosphorylase (PNP), and the resultant hypoxanthine is oxidized by xanthine oxidase (XOD) to produce uric acid. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye, which is colorimetrically assessed.

### (1-2)

Pyruvic acid is oxidized by pyruvic oxidase (POP) in the presence of inorganic phosphorus (Pi), cocarboxylase (TPP), flavin adenine dinucleotide (FAD) and Mg²⁺ to produce acetyl acetate. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye which is colorimetrically assessed, in the same manner as described in (1-1).

It is noted that the last color reaction for each of the above processes (1-1) and (1-2) can be performed by a "Trinder reagent" which is known as a detection reagent for hydrogen peroxide. In this reaction, phenols function as "hydrogen donors." Phenols to be used as "hydrogen donors" are classical, and now various modified "hydrogen donors" are used. Examples of these hydrogen donors include N-ethyk-N-sulfopropyl-m-anilidine, N-ethyl-N-sulfopropylaniline, N-ethyl-N-sulfopropyl-3,5-dimethoxyaniline, N-sulfopropyl-3,5-dimethoxyaniline, N-ethyl-N-sulfopropyl-3,5-dimethylaniline, N-ethyl-N-sulfopropyl-m-toluidine, N-ethyl-N-(2-hydroxy-3-sulfopropyl)-m-anilidine N-ethyl-N-(2-hydroxy-3-sulfopropyl)aniline, N-ethyl-N-(2-hydroxy-3-sulfopropyl)-3,5-dimethoxyaniline, N-(2-hydroxy-3-sulfopropyl)-3,5-dimethoxyaniline, N-ethyl-N-(2-hydroxy-3-sulfopropyl)-3,5-dimethylaniline, N-ethyl-N-(2-hydroxy-3-sulfopropyl)-m-toluidine, and N-sulfopropylaniline.

### (2) Example of a method using glucose-6-phosphate dehydrogenase (G6PDH)

### (2-1)

Inorganic phosphorus (Pi) is reacted with glycogen with phosphorylase to produce glucose-1-phosphate (G-1-P). The produced glucose-1-phosphate is converted into glucose-6-phosphate (G-6-P) with phosphoglucomutase (PGM). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### (2-2)

Inorganic phosphorus (Pi) is reacted with maltose with maltose phosphorylase (MP) to produce glucose-1-phosphate (G-1-P). Thereafter, the produced glucose-1-phosphate is converted into glucose-6-phosphate (G-6-P) with phosphoglucomutase (PGM) in the same manner as described in (2-1). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### B. Phosphomolybdate method

There are two phosphomolybdate methods. One is a direct method wherein "Phosphomolybdates (H₃[PO₄Mo₁₂O₃₆])" prepared by complexing inorganic phosphorus (phosphate) and aqueous molybdate ions under acidic condition are directly quantified. The other is a reduction method wherein further to the above direct method, Mo(IV) is reduced to Mo(III) by a reducing agent and molybudenum blue (Mo(III)) is quantified. Examples of the aqueous molybdate ions include aluminum molybdate, cadmium molybdate, calcium molybdate, barium molybdate, lithium molybdate, potassium molybdate, sodium molybdate, and ammonium molybdate. Representative examples of the reducing agents to be used in the reduction method include 1-amino-2-naphthol-4-sulfonic acid, ammonium ferrous sulfate, ferrous chloride, stannous chloride-hydrazine, p-methylaminophenol sulfate, N,N-dimethyl-phenylenediamine, ascorbic acid, and malachite green.

When a light-transmissive and water-impervious support is used, the dry analytical element can be practically constructed as below. However, the scope of the present invention is not limited to these.
(1) One having a reagent layer on the support.
(2) One having a detection layer and a reagent layer in that order on the support.
(3) One having a detection layer, a light reflection layer, and a reagent layer in that order on the support.
(4) One having a second reagent layer, a light reflection layer, and a first reagent layer in that order on the support.
(5) One having a detection layer, a second reagent layer, a light reflection layer, and a first reagent layer in that order on the support.

In (1) to (3) above, the reagent layer may be constituted by a plurality of different layers. For example, a first reagent layer may contain enzyme pyrophosphatase which is required in the pyrophosphatase reaction represented by formula 2 or 3, and substrate xanthosine or substrate inosine and enzyme PNP which are required in the PNP reaction, a second reagent layer may contain enzyme XOD which is required in the XOD reaction represented by formula 2 or 3, and a third reagent layer may contain enzyme POD which is required in the POD reaction represented by formula 2 or 3, and a coloring dye (dye precursor). Alternatively, two reagent layers are provided. On the first reagent layer, the pyrophosphatase reaction and the PNP reaction may be proceeded, and the XOD reaction and the POD reaction may be proceeded on the second reagent layer. Alternatively, the pyrophosphatase reaction, the PNP reaction and the XOD reaction may be proceeded on the first reagent layer, and the POD reaction may be proceeded on the second reagent layer.

A water absorption layer may be provided between a support and a reagent layer or detection layer. A filter layer may be provided between each layer. A developing layer may be provided on the reagent layer and an adhesive layer may be provided therebetween.

Any of light-nontransmissive (opaque), light-semitransmissive (translucent), or light-transmissive (transparent) support can be used. In general, a light-transmissive and water-impervious support is preferred. Preferable materials for a light-transmissive and water-impervious support are polyethylene terephthalate or polystyrene. In order to firmly adhere a hydrophilic layer, an undercoating layer is generally provided or hydrophilization is carried out.

When a porous layer is used as a reagent layer, the porous medium may be a fibrous or nonfibrous substance. Fibrous substances used herein include, for example, filter paper, non-woven fabric, textile fabric (e.g. plain-woven fabric), knitted fabric (e.g., tricot knitted fabric), and glass fiber filter paper. Nonfibrous substances may be any of a membrane filter comprising cellulose acetate etc., described in Japanese Patent Publication Laying-Open No. 49-53888 and the like, or a particulate structure having mutually interconnected spaces comprising fine particles of inorganic substances or organic substances described in, for example, Japanese Patent Publication Laying-Open No. 49-53888, Japanese Patent Publication Laying-Open No. 55-90859 (U.S. Patent. No. 4,258,001), and Japanese Patent Publication Laying-Open No. 58-70163 (U.S. Patent. No. 4,486,537). A partially-adhered laminate which comprises a plurality of porous layers described in, for example, Japanese Patent Publication Laying-Open No. 61-4959 (EP Publication 0166365A), Japanese Patent Publication Laying-Open No. 62-116258, Japanese Patent Publication Laying-Open No. 62-138756 (EP Publication 0226465A), Japanese Patent Publication Laying-Open No. 62-138757 (EP Publication 0226465A), and Japanese Patent Publication Laying-Open No. 62-138758 (EP Publication 0226465A), is also preferred.

A porous layer may be a developing layer having so-called measuring action, which spreads liquid in an area substantially in proportion to the amount of the liquid to be supplied. Preferably, a developing layer is textile fabric, knitted fabric, and the like. Textile fabrics and the like may be subjected to glow discharge treatment as described in Japanese Patent Publication Laying-Open No. 57-66359. A developing layer may comprise hydrophilic polymers or surfactants as described in Japanese Patent Publication Laying-Open No. 60-222770 (EP 0162301A), Japanese Patent Publication Laying-Open No. 63-219397 (German Publication DE 3717913A), Japanese Patent Publication Laying-Open No. 63-112999 (DE 3717913A), and Japanese Patent Publication Laying-Open No. 62-182652 (DE 3717913A) in order to regulate a developing area, a developing speed and the like.

For example, a method is useful where the reagent of the present invention is previously impregnated into or coated on a porous membrane etc., comprising paper, fabric or polymer, followed by adhesion onto another water-pervious layer provided on a support (e.g., a detection layer) by the method as described in Japanese Patent Publication Laying-Open No. 55-1645356.

The thickness of the reagent layer thus prepared is not particularly limited. When it is provided as a coating layer, the thickness is suitably in the range of about 1 µm to 50 µm, preferably in the range of 2 µm to 30 µm. When the reagent layer is provided by a method other than coating, such as lamination, the thickness can be significantly varied in the range of several tens of to several hundred µm.

When a reagent layer is constituted by a water-pervious layer of hydrophilic polymer binders, examples of hydrophilic polymers which can be used include: gelatin and a derivative thereof (e.g., phthalated gelatin); a cellulose derivative (e.g., hydroxyethyl cellulose); agarose, sodium arginate; an acrylamide copolymer or a methacrylamide copolymer (e.g., a copolymer of acrylamide or methacrylamide and various vinyl monomers); polyhydroxyethyl methacrylate; polyvinyl alcohol; polyvinyl pyrrolidone; sodium polyacrylate; and a copolymer of acrylic acid and various vinyl monomers.

A reagent layer composed of hydrophilic polymer binders can be provided by coating an aqueous solution or water dispersion containing the reagent composition of the present invention and hydrophilic polymers on the support or another layer such as a detection layer followed by drying the coating in accordance with the methods described in the specifications of Japanese Patent Examined Publication No. 53-21677 (U.S. Patent No. 3,992,158), Japanese Patent Publication Laying-Open No. 55-164356 (U.S. Patent. No. 4,292,272), Japanese Patent Publication Laying-Open No. 54-101398 (U.S. Patent. No. 4,132,528) and the like. The thickness of the reagent layer comprising hydrophilic polymers as binders is about 2 µm to about 50 µm, preferably about 4 µm to about 30 µm on a dry basis, and the coverage is about 2 g/m² to about 50 g/m², preferably about 4 g/m² to about 30 g/m².

The reagent layer can further comprise an enzyme activator, a coenzyme, a surfactant, a pH buffer composition, an impalpable powder, an antioxidant, and various additives comprising organic or inorganic substances in addition to the reagent composition represented by formula 2 or 3 in order to improve coating properties and other various properties of diffusible compounds such as diffusibility, reactivity, and storage properties. Examples of buffers which can be contained in the reagent layer include pH buffer systems described in "Kagaku Binran Kiso (Handbook on Chemistry, Basic)," The Chemical Society of Japan (ed.), Maruzen Co., Ltd. (1996), p.1312-1320, "Data for Biochemical Research, Second Edition, R. M. C. Dawson et al. (2nd ed.), Oxford at the Clarendon Press (1969), p. 476-508, "Biochemistry" 5, p. 467-477 (1966), and "Analytical Biochemistry" 104, p. 300-310 (1980). Specific examples of pH buffer systems include a buffer containing borate; a buffer containing citric acid or citrate; a buffer containing glycine, a buffer containing bicine; a buffer containing HEPES; and Good's buffers such as a buffer containing MES. A buffer containing phosphate cannot be used for a dry analytical element for detecting pyrophosphoric acid.

The dry analytical element for quantifying pyrophosphoric acid which can be used in the present invention can be prepared in accordance with a known method disclosed in the above-described various patent specifications. The dry analytical element for quantifying pyrophosphoric acid is cut into small fragments, such as, an about 5 mm to about 30 mm-square or a circle having substantially the same size, accommodated in the slide frame described in, for example, Japanese Patent Examined Publication No. 57-283331 (U.S. Patent. No. 4,169,751), Japanese Utility Model Publication Laying-Open No. 56-142454 (U.S. Patent. No. 4,387,990), Japanese Patent Publication Laying-Open No. 57-63452, Japanese Utility Model Publication Laying-Open No. 58-32350, and Japanese Patent Publication Laying-Open No. 58-501144 (International Publication WO 083/00391), and used as slides for chemical analysis. This is preferable from the viewpoints of production, packaging, transportation, storage, measuring operation, and the like. Depending on its intended use, the analytical element can be accommodated as a long tape in a cassette or magazine, as small pieces accommodated in a container having an opening, as small pieces applied onto or accommodated in an open card, or as small pieces cut to be used in that state.

The dry analytical element for quantifying pyrophosphoric acid which can be used in the present invention can quantitatively detect pyrophosphoric acid which is a test substance in a liquid sample, by operations similar to that described in the above-described patent specifications and the like. For example, about 2 µL to about 30 µL, preferably 4 µL to 1 µL of aqueous liquid sample solution is spotted on the reagent layer. The spotted analytical element is incubated at constant temperature of about 20°C to about 45°C, preferably about 30°C to about 40°C for 1 to 10 minutes. Coloring or discoloration in the analytical element is measured by the reflection from the light-transmissive support side, and the amount of pyrophosphoric acid in the specimen can be determined based on the principle of colorimetry using the previously prepared calibration curve. Quantitative analysis can be carried out with high accuracy by keeping the amount of liquid sample to be spotted, the incubation time, and the temperate at constant levels.

Quantitative analysis can be carried out with high accuracy in a very simple operation using chemical analyzers described in, for example, Japanese Patent Publication Laying-Open No. 60-125543, Japanese Patent Publication Laying-Open No. 60-220862, Japanese Patent Publication Laying-Open No. 61-294367, and Japanese Patent Publication Laying-Open No. 58-161867 (U.S. Patent. No. 4,424,191). Semiquantitative measurement may be carried out by visually judging the level of coloring depending on the purpose and accuracy needed.

Since the dry analytical element for quantifying pyrophosphoric acid which can be used in the present invention is stored and kept in a dry state before analysis, it is not necessary that a reagent is prepared for each use, and stability of the reagent is generally higher in a dry state. Thus, in terms of simplicity and swiftness, it is better than a so-called wet process, which requires the preparation of the reagent solution for each use. It is also excellent as an examination method because highly accurate examination can be swiftly carried out with a very small amount of liquid sample.

The dry analytical element for quantifying inorganic phosphorus which can be used in the second aspect of the present invention can be prepared by removing pyrophosphatase from the reagent layer in the aforementioned dry analytical element for quantifying pyrophosphoric acid. The dry analytical element described in Japanese Patent Publication Laying-Open No. 7-197 can also be used. The dry analytical element for quantifying inorganic phosphorus is similar to the aforementioned dry analytical element for quantifying pyrophosphoric acid in its layer construction, method of production, and method of application, with the exception that the reagent layer does not comprise pyrophosphatase.

The present invention is described in more detail with reference to the following examples.

### Examples

### Example 1: Detection of the genus Mycobacterium (acid-fast bacteria) using pyrophosphoric acid slide (performance confirmation using cultured bacterial strain)

### (1) Sample preparation

Cultured bacterial samples that had been previously identified as being of the bacterial species *Mycobacterium tuberculosis* (Mtb), *Mycobacterium avium* (Ma), or *Mycobacterium intracellulare* (Mi) were prepared. After washing the harvested bacteria, genomic DNA was extracted according to R. Boom et al's method (Journal of Clinical Microbiology vol. 28, No. 3, p. 495 (1990)).

### (2) PCR amplification reaction

A PCR amplification reaction was performed using the DNA solution prepared in (1) above under the following conditions.

### <Primer>

t2 (upper: for Mtb detection): 5'-ataccggataggaccacg-3' (SEQ ID NO: 10)
a2 (upper: for Ma detection): 5'-ataccggataggacctca-3' (SEQ ID NO: 11)
i2 (upper: for Mi detection): 5'-aataccggataggaccttt-3' (SEQ ID NO: 12)
M2 (lower: common among all 3 bacterial species): 5'-tgcttcttctccacctacc-3'(SEQ ID NO: 13)

The PCR reaction was performed with combinations of primers for detecting each bacterium and specimens listed in Table 1 below.

**Table 1:**

| PCR primer (upper/lower) | | Specimen type | | |
|---|---|---|---|---|
| | Mtb | Ma | Mi | Negative control |
| t2/M2 | (1) | (2) | (3) | (4) |
| a2/M2 | (5) | (6) | (7) | (8) |
| i2/M2 | (9) | (10) | (11) | (12) |
| | | Series 1 | | Series 2 |

As listed above, for series 1 ((1) to (3), (5) to (7), and (9) to (11)) and series 2 ((4), (8), and (12)), a PCR amplification reaction was implemented by repeating 40 cycles of reaction [denaturation: 94°C for 15 seconds; annealing: 63°C for 30 seconds; and polymerase elongation reaction: 72°C for 30 seconds] with the reaction solution composition shown in Table 2 below.

**Table 2: <Reaction solution composition>**

| | Series 1 | Series 2 (negative control) |
|---|---|---|
| 10×PCR buffer | 5 µL | 5 µL |
| 2.5 mM dNTP | 4 µL | 4 µL |
| 5 µM primer (upper) | 2.5 µL | 2.5 µL |
| 5 µM primer (lower) | 2.5 µL | 2.5 µL |
| HS Taq (produced by TAKARA BIO INC.) | 0.5 µL | 0.5 µL |
| Each nucleic acid solution obtained in (1) | 1 µL | 0 µL |
| Purified water | 34.5 µL | 35.5 µL |
| Total | 50 µL | 50 µL |

### (3) Production of dry analytical element for quantifying pyrophosphoric acid

An aqueous solution of composition (a) described in Table 3 below was applied onto a 180-µm-thick transparent and colorless polyethylene terephthalate (PET) smooth film sheet (support) provided with gelatin undercoat, so as to have the following coating ratios. The resultant was then dried so as to provide a reagent layer.

**Table 3: Composition (a) of the aqueous solution applied onto the reagent layer**

| | |
|---|---|
| Gelatin | 18.8 g/m² |
| p-nonylphenoxy polyxydol (glycidol units: contained 10 on average) (C₉H₁₉-Ph-O-(CH₂CH(OH)-CH₂-O)₁₀H) | 1.5 g/m² |
| Xanthosine | 1.96 g/m² |
| Peroxidase | 15000 IU/m² |
| Xanthine oxidase | 13600 IU/m² |
| Purine nucleoside phosphorylase | 3400 IU/m² |
| Leuco pigment (2-(3,5-dimethoxy-4-hydroxyphenyl)-4-phenethyl-5-(4-dimethylaminophenyl) imidazole) | 0.28 g/m² |
| Water | 136 g/m² |

(pH was adjusted to 6.8 using a dilute NaOH solution)

An aqueous solution of an adhesion layer, which has a composition (b) described in Table 4 below, was applied onto the reagent layer so as to have the following coating ratios. The resultant was then dried, so as to provide the adhesion layer.

**Table 4: Composition (b) of the aqueous solution applied onto the adhesion layer**

| | |
|---|---|
| Gelatin | 3.1 g/m² |
| p-nonylphenoxy polyxydol (glycidol units: contained 10 on average) (C₉H₁₉-Ph-O-(CH₂CH(OH)-CH₂-O)₁₀H) | 0.25 g/m² |
| Water | 59 g/m² |

Subsequently, water was supplied at a rate of 30 g/m² over the entire surface of the adhesion layer, thereby causing the gelatin layer to swell. A broadcloth textile made of pure polyester was applied almost uniformly as lamination by applying slight pressure thereto, thereby providing a porous development layer.

Next, an aqueous solution with a composition (c) described in Table 5 below was applied almost uniformly onto the development layer, so as to have the following coating ratios. The resultant was dried and then cut into pieces with the size of 13 mm × 14 mm. The resultant was contained in a plastic mounting material, and thus, a dry analytical element for quantifying pyrophosphoric acid was produced.

**Table 5: Composition (c) of the aqueous solution applied onto the development layer**

| | |
|---|---|
| HEPES | 2.3 g/m² |
| Sucrose | 5.0 g/m² |
| Hydroxypropylmethylcellulose | 0.04 g/m² |
| (methoxy groups: 19% to 24%; hydroxy propoxy groups 4% to 12%) | |
| Pyrophosphatase | 14000 IU/m² |
| Water | 98.6 g/m² |

(pH was adjusted to 7.2 using a dilute NaOH solution)

### (4) Detection using analytical element for quantifying pyrophosphoric acid

20 µL of the solution obtained after the PCR amplification reaction in (2) above was placed directly on each dry analytical element for quantifying pyrophosphoric acid produced in (3) above. After 5 minutes of incubation of the dry analytical elements for quantifying pyrophosphoric acid at 37°C, reflection optical density (OD_{R}) was measured after 5 minutes of incubation from the support side at a wavelength of 650 nm. Table 6 shows such reflection optical densities and the numerical values of the same represented by pyrophosphoric acid concentrations (mM) based on a calibration curve that had been previously prepared to convert reflection optical densities into pyrophosphoric acid concentrations.

**Table 6: Relationship between the initial template amount in PCR reaction and reflection optical density (OD_{R}) after 5 minutes**

| Amplification sample No. | | Reflection optical density (OD_{R}) after 5 minutes | Pyrophosphoric acid concentration (mM) |
|---|---|---|---|
| M.tb | (1) | 0.647 | 0.105 |
| | (2) | 0.490 | 0.044 |
| | (3) | 0.464 | 0.036 |
| | (4) | 0.475 | 0.039 |
| M.a | (5) | 0.472 | 0.038 |
| | (6) | 0.566 | 0.071 |
| | (7) | 0.461 | 0.035 |
| | (8) | 0.484 | 0.042 |
| M.i | (9) | 0.460 | 0.034 |
| | (10) | 0.464 | 0.036 |
| | (11) | 0.589 | 0.080 |
| | (12) | 0.479 | 0.041 |

As shown in the underlined results in Table 6, the PCR amplification reaction was performed for genomes derived from each bacterium using primers for detecting each acid-fast bacterium. The generated pyrophosphoric acid was quantified by measuring reflection optical density (OD_{R}) using the solution directly after the PCR amplification reaction and using dry analytical elements for quantifying pyrophosphoric acid. Thus, only the bacteria corresponding to the primers for detecting each bacterial species could be specifically detected.

### Example 2: Detection of the genus Mycobacterium (acid-fast bacteria) using pyrophosphoric acid slide (performance confirmation using cultured bacterial strain)

### (1) Sample preparation

Cultured bacterial samples that had been previously identified as being of the bacterial species *M. tuberculosis* (Mtb), *M. avium* (Ma), *M. intaracellulare* (Mi), or *M. kansasii* (Mk) were prepared. After washing the harvested bacteria, genomic DNA was extracted according to R. Boom et al's method (Journal of Clinical Microbiology vol. 28, No. 3, p. 495 (1990)).

### (2) PCR amplification reaction

A PCR amplification reaction was performed using the DNA solution prepared in (1) above under the following conditions.

### <Primer>

k (upper: Mk detection): 5'- ataccggataggaccacttg -3'(SEQ ID NO: 26)
M5 (lower): 5'- cgtcctgtgcatgtcaaa -3'(SEQ ID NO: 28)

The PCR reaction was performed with combinations of primers for detecting each bacterium and specimens as listed below.

**Table 7:**

| PCR primer (upper/lower) | Specimen type | | | | |
|---|---|---|---|---|---|
| | M. tb | M. a | M. i | M. k | Negative control |
| k/M5 | (1) | (2) | (3) | (4) | (5) |

As listed above, for (1) to (5), a PCR amplification reaction was implemented by repeating 40 cycles of reaction [denaturation: 94°C for 15 seconds; annealing: 63°C for 30 seconds; and polymerase elongation reaction: 72°C for 30 seconds] with the reaction solution composition shown below.

**Table 8: <Reaction solution composition>**

| | Series 1 | Series 2 (negative control) |
|---|---|---|
| 10×PCR buffer | 5 µL | 5 µL |
| 2.5 mM dNTP | 4 µL | 4 µL |
| 5 µM primer (upper) | 2.5 µL | 2.5 µL |
| 5 µM primer (lower) | 2.5 µL | 2.5 µL |
| HS Taq (produced by TAKARA BIO INC.) | 0.5 µL | 0.5 µL |
| Each nucleic acid solution obtained in (1) | 1 µL | 0 µL |
| Purified water | 34.5 µL | 33.5 µL |
| Total | 50 µL | 50 µL |

### (3) Production of dry analytical element for quantifying pyrophosphoric acid

An aqueous solution of composition (a) described in Table 9 below was applied onto a 180-µm-thick transparent and colorless polyethylene terephthalate (PET) smooth film sheet (support) provided with gelatin undercoat so as to have the following coating ratios. The resultant was then dried so as to provide a reagent layer.

**Table 9: Composition (a) of the aqueous solution applied onto the reagent layer**

| | |
|---|---|
| Gelatin | 18.8 g/m² |
| p-nonylphenoxy polyxydol (glycidol units: contained 10 on average) (C₉H₁₉-Ph-O-(CH₂CH(OH)-CH₂-O)₁₀H) | 1.5 g/m² |
| Xanthosine | 1.96 g/m² |
| Peroxidase | 15000 IU/m² |
| Xanthine oxidase | 13600 IU/m² |
| Purine nucleoside phosphorylase | 3400 IU/m² |
| Leuco pigment (2-(3,5-dimethoxy-4-hydroxyphenyl)-4-phenethyl-5-(4-dimethylaminophenyl) imidazole) | 0.28 g/m² |
| Water | 136 g/m² |

(pH was adjusted to 6.8 using a dilute NaOH solution)

An aqueous solution of an adhesion layer, which has a composition (b) described in Table 10 below, was applied onto the reagent layer so as to have the following coating ratios. The resultant was then dried, so as to provide the adhesion layer.

**Table 10: Composition (b) of the aqueous solution applied onto the adhesion layer**

| | |
|---|---|
| Gelatin | 3.1 g/m² |
| p-nonylphenoxy polyxydol (glycidol units: contained 10 on average) (C₉H₁₉-Ph-O-(CH₂CH(OH)-CH₂-O)₁₀H) | 0.25 g/m² |
| Water | 59 g/m² |

Subsequently, water was supplied at a rate of 30 g/m² over the entire surface of the adhesion layer, thereby causing the gelatin layer to swell. A broadcloth textile made of pure polyester was applied almost uniformly as lamination by applying slight pressure thereto, thereby providing a porous development layer.

Next, an aqueous solution with a composition (c) described in Table 11 below was applied almost uniformly onto the development layer, so as to have the following coating concentrations. The resultant was dried and then cut into pieces with the size of 13 mm × 14 mm. The resultant was contained in a plastic mounting material, and thus a dry analytical element for quantifying pyrophosphoric acid was produced.

**Table 11: Composition (c) of the aqueous solution applied onto the development layer**

| | |
|---|---|
| HEPES | 2.3 g/m² |
| Sucrose | 5.0 g/m² |
| Hydroxypropylmethylcellulose | 0.04 g/m² |
| (methoxy groups: 19% to 24%; hydroxy propoxy groups 4% to 12%) | |
| Pyrophosphatase | 14000 IU/m² |
| Water | 98.6 g/m² |

(pH was adjusted to 7.2 using a dilute NaOH solution)

### (4) Detection using analytical element for quantifying pyrophosphoric acid

20 µL of the solution obtained after the PCR amplification reaction in (2) above was placed directly on each dry analytical element for quantifying pyrophosphoric acid produced in (3) above. After 5 minutes of incubation of the dry analytical elements for quantifying pyrophosphoric acid at 37°C, reflection optical density (OD_{R}) was measured after 5 minutes of incubation from the support side at a wavelength of 650 nm. Table 12 shows such reflection optical densities and the numerical values of the same represented by pyrophosphoric acid concentrations (mM) based on a calibration curve that had been previously prepared to convert reflection optical densities into pyrophosphoric acid concentrations.

**Table 12: Relationship between the initial template amount in PCR reaction and reflection optical density (OD_{R}) after 5 minutes**

| Amplification sample No. | | Reflection optical density (OD_{R}) after 5 minutes | Pyrophosphoric acid concentration (mM) |
|---|---|---|---|
| M. k | (1) | 0.449 | 0.003 |
| | (2) | 0.439 | 0.000 |
| | (3) | 0.435 | 0.000 |
| | (4) | 0.748 | 0.124 |
| | (5) | 0.441 | |

As shown in the results in Example 2, the PCR amplification reaction was performed for genomes derived from each bacterium using primers for detecting *M. kansasii*. The generated pyrophosphoric acid was quantified by measuring reflection optical density (OD_{R}) using the solution directly after the PCR amplification reaction and using dry analytical elements for quantifying pyrophosphoric acid. Thus, only *M*. *kansasii* could be specifically detected by the use of the primers for detecting the bacterial species of *M. kansasii*.

### INDUSTRIAL APPLICABILITY

The present invention has enabled rapid and convenient detection of bacteria of the genus Mycobacterium (acid-fast bacteria) or identification of the bacterial species thereof.

## Claims

1. A method for identifying *Mycobacterium tuberculosis,* which comprises performing a nucleic acid amplification reaction using, as primers, an upper primer having a nucleotide sequence represented by SEQ ID NO: 10 and a lower primer represented by SEQ ID NO: 13.

2. A method for identifying *Mycobacterium avium,* which comprises performing a nucleic acid amplification reaction using, as primers, an upper primer having a nucleotide sequence represented by SEQ ID NO: 11 and a lower primer represented by SEQ ID NO: 13.

3. A method for identifying *Mycobacterium intracellulare,* which comprises performing a nucleic acid amplification reaction using, as primers, an upper primer having a nucleotide sequence represented by SEQ ID NO: 12 and a lower primer represented by SEQ ID NO: 13.

4. A method for identifying *Mycobacterium kansasii,* which comprises performing a nucleic acid amplification reaction using, as primers, an upper primer having a nucleotide sequence represented by SEQ ID NO: 26 and a lower primer represented by SEQ ID NO: 28.

5. The method according to any one of claims 1 to 4, which comprises detecting a by-product of a nucleic acid amplification reaction.

6. The method according to claim 5, wherein the by-product of the nucleic acid amplification reaction is pyrophosphoric acid.

7. The method according to claim 6, wherein pyrophosphoric acid is detected using a dry analytical element.

## Patentansprüche

1. Verfahren zum Identifizieren von *Mycobacterium tuberculosis,* das Durchführen einer Nukleinsäure-Amplifikationsreaktion unter Verwendung als Primer eines oberen Primers, der eine durch SEQ ID NO:10 dargestellte Nukleotidsequenz hat, und eines durch SEQ ID NO:13 dargestellten unteren Primers umfasst.

2. Verfahren zum Identifizieren von *Mycobacterium avium,* das Durchführen einer Nukleinsäure-Amplifikationsreaktion unter Verwendung als Primer eines oberen Primers, der eine durch SEQ ID NO:11 dargestellte Nukleotidsequenz hat, und eines durch SEQ ID NO:13 dargestellten unteren Primers umfasst.

3. Verfahren zum Identifizieren von *Mycobacterium intracellulare,* das Durchführen einer Nukleinsäure-Amplifikations-reaktion unter Verwendung als Primer eines oberen Primers, der eine durch SEQ ID NO:12 dargestellte Nukleotidsequenz hat, und eines durch SEQ ID NO:13 dargestellten unteren Primers umfasst.

4. Verfahren zum Identifizieren von *Mycobacterium kansasii,* das Durchführen einer Nukleinsäure-Amplifikationsreaktion unter Verwendung als Primer eines oberen Primers, der eine durch SEQ ID NO:26 dargestellte Nukleotidsequenz hat, und eines durch SEQ ID NO:28 dargestellten unteren Primers umfasst.

5. Verfahren nach einem der Ansprüche 1 bis 4, welches Detektieren eines Nebenprodukts einer Nukleinsäure-Amplifikationsreaktion umfasst.

6. Verfahren nach Anspruch 5, wobei das Nebenprodukt der Nukleinsäure-Amplifikationsreaktion Pyrophosphorsäure ist.

7. Verfahren nach Anspruch 6, wobei Pyrophosphorsäure unter Verwendung eines Trockenanalyseelements detektiert wird.

## Revendications

1. Procédé d'identification de *Mycobacterium tuberculosis* comprenant l'étape consistant à effectuer une réaction d'amplification d'acide nucléique en utilisant, comme amorces, une amorce supérieure ayant une séquence de nucléotide représentée par la SEQ ID NO: 10 et une amorce inférieure représentée par la SEQ ID NO: 13.

2. Procédé d'identification de *Mycobacterium tuberculosis* comprenant l'étape consistant à effectuer une réaction d'amplification d'acide nucléique en utilisant, comme amorces, une amorce supérieure ayant une séquence de nucléotide représentée par la SEQ ID NO: 11 et une amorce inférieure représentée par la SEQ ID NO: 13.

3. Procédé d'identification de *Mycobacterium tuberculosis* comprenant l'étape consistant à effectuer une réaction d'amplification d'acide nucléique en utilisant, comme amorces, une amorce supérieure ayant une séquence de nucléotide représentée par la SEQ ID NO: 12 et une amorce inférieure représentée par la SEQ ID NO: 13.

4. Procédé d'identification de *Mycobacterium tuberculosis* comprenant l'étape consistant à effectuer une réaction d'amplification d'acide nucléique en utilisant, comme amorces, une amorce supérieure ayant une séquence de nucléotide représentée par la SEQ ID NO: 26 et une amorce inférieure représentée par la SEQ ID NO: 28.

5. Procédé selon l'une quelconque des revendications 1 à 4 comprenant l'étape consistant à détecter un produit secondaire d'une réaction d'amplification d'acide nucléique.

6. Procédé selon la revendication 5, dans lequel le produit secondaire de la réaction d'amplification d'acide nucléique est l'acide pyrophosphorique.

7. Procédé selon la revendication 6, dans lequel l'acide pyrophosphorique est détecté en utilisant un élément analytique sec.
